# EUROPÄISCHE PATENTSCHRIFT

(11) **EP 3 513 169 B1**
(45) Veröffentlichungstag und Bekanntmachung des Hinweises auf die Patenterteilung: **24.06.2020**
(21) Anmeldenummer: 17758538.7
(22) Anmeldetag: 28.08.2017
(51) Int. Cl.: G01N 21/15, G01N 21/51, G01N 33/18

(54) **WASSERANALYSE-GERÄT**
WATER ANALYZER
DISPOSITIF D'ANALYSE D'EAU

(30) Priorität: 15.09.2016 DE 202016105143 U
(43) Veröffentlichungstag der Anmeldung: 24.07.2019
(73) Patentinhaber: Hach Lange GmbH, 14163 Berlin (DE)
(72) Erfinder: KUMPCH, Hans-Joachim, 12207 Berlin (DE); DRAEGER, Hartmut, 10781 Berlin (DE); LIEBCHEN, Detlef, 13437 Berlin (DE)
(74) Vertreter: Sandri, Sandro
(86) Internationale Anmeldenummer: PCT/EP2017/071568
(87) Internationale Veröffentlichungsnummer: WO 2018/050428

(56) Entgegenhaltungen:
- EP-A2- 0 109 631
- WO-A1-2016/079259
- JP-A- 2006 337 106
- US-A- 5 446 544

## Beschreibung

Die Erfindung bezieht sich auf ein optisches Wasseranalyse-Gerät mit einer Lichtquelle und einem Licht-Detektor zur Erfassung eines optischen Parameters einer Wasserprobe in einer transparenten Meßküvette. Die Erfindung bezieht sich insbesondere auf ein Prozess-Trübungsmessgerät.

Ein gattungsgemäßes Wasseranalyse-Gerät ist bekannt aus WO 2016/079259 A1. Das Wasseranalyse-Gerät weist eine Küvettenkammer auf, in der eine Meßküvette angeordnet ist. Das Wasseranalyse-Gerät weist ferner einen Lüftungskreis zum Belüfteten der Küvettenkammer auf, wodurch die Kondensatbildung insbesondere an der Meßküvette verhindert werden soll. Der Lüftungskreis weist eine Belüftung-Pumpe stromabwärts der Küvettenkammer auf. Die Küvettenkammer weist einen Lufteinlass stromabwärts der Belüftung-Pumpe auf. Es ist ein Gerätegehäuse vorgesehen, das die Küvettenkammer bildet, und es ist eine Deckelanordnung vorgesehen, die die Küvettenkammer fluidisch gegenüber der Atmosphäre verschließt bzw. abschirmt. Die fluiddichte Abdichtung der Deckelanordnung gegenüber dem Gerätegehäuse ist wichtig, damit keine Luft bzw. mit der Luft kein Staub und keine Luftfeuchtigkeit in die Küvettenkammer bzw. in das Geräteinnere eindringen, da hierdurch die Streuungsmessung empfindlich gestört wird. Die Deckelanordnung muss leicht zu öffnen sein, damit ein einfacher Zugriff auf die Küvettenkammer und die Meßküvette möglich ist.

Aus der EP 0 109 631 A2 ist ein Schraubverschluss bekannt, der eine lange dünne Dichtlippe aufweist, die sich vom Ringteil des Verschlusses gegen die Behältermündung und deren Aussenrand erstreckt. In der letzten Phase des Aufschraubens wird sie von einer am Kappenteil angebrachten Druckrippe auf die Mündung aufgepresst. Weil die Wurzel der Dichtlippe sich jedoch noch weiter nach unten bewegt, andererseits die Berührungsstelle zwischen Druckrippe und Dichtlippe an Ort und Stelle verbleibt, wird der dazwischenliegende Abschnitt gedehnt. Er legt sich daher unter Druck gegen die Mündung und insbesondere gegen den Aussenrand des Behälters an.

Die US 5 446 544 A offenbart ein Wasseranalyse-Gerät mit einer transparenten Messküvette, einer Lichtquelle und einem Lichtdetektor zur Erfassung eines optischen Parameters (Trübung) einer Wasserprobe, einem Gehäuse, das eine Küvettenkammer bildet, in der die Messküvette angeordnet ist, einer Deckelanordnung, mit der die Küvettenkammer fluidisch verschlossen ist, und einem Ventilator zur Belüftung der Küvettenkammer. Die Deckelanordnung weist ein inneres Gewinde auf, in das die Messküvette eingeschraubt ist, und wird mittels eines Joch-Elements am Gehäuse fixiert. Die Abdichtung erfolgt mittels eines O-Rings.

Aufgabe der Erfindung ist es, ein Wasseranalyse-Gerät mit einer Deckelanordnung zu schaffen, die eine zuverlässige Abdichtung und eine leichte Bedienbarkeit bietet.

Diese Aufgabe wird erfindungsgemäß gelöst mit einem Wasseranalyse-Gerät mit den Merkmalen des Anspruchs 1.

Das Gerätegehäuse bildet und definiert unter anderem die Küvettenkammer, in der im Messbetrieb die Meßküvette angeordnet ist. Die Deckelanordnung und das Gerätegehäuse weisen eine Drehverschluss-Mimik auf, so dass die Deckelanordnung durch eine einfache Drehbewegung geschlossen und ggf. an dem Gerätegehäuse verriegelt bzw. geöffnet ggf. entriegelt und werden kann. Die Drehbewegung kann eine Schraubbewegung sein, kann jedoch auch eine im Wesentlichen rein zirkuläre Drehbewegung sein. In jedem Fall wird durch die schließende Drehbewegung eine Festsetzung bzw. Verriegelung der Deckelanordnung an dem Gerätegehäuse hergestellt. Eine Drehbewegung ist einfach vornehmbar von einer Bedienperson, und bei einer Drehbewegung können durch die Bedienperson relativ hohe Drehkräfte für die Schließbewegung bzw. die Öffnungsbewegung aufgebracht werden. Auf diese Weise lässt sich insbesondere ein werkzeugloses Verschließen und öffnen der Deckelanordnung an dem Gerätegehäuse realisieren. Hierdurch wird eine einfache Handhabung insbesondere bei Wartungsarbeiten sichergestellt.

Das Gerätegehäuse einerseits und die Deckelanordnung andererseits bilden und definieren zusammen eine kreisringförmige und zu der Drehbewegung koaxiale Ringdichtung, die bei geschlossener und verriegelter Deckelanordnung eine fluiddichte Abdichtung des Geräte-Innenraums sicherstellen soll.

Die Ringdichtung wird von einem flexiblen und elastischen Dichtungskörper mit einer zirkulären Dichtlippe einerseits und einem korrespondierenden zirkulären Schultersitz andererseits gebildet. Bei Betrieb der Belüftungs-Pumpe herrscht in der Küvettenkammer stets ein gewisser Unterdruck gegenüber dem außenseitigen Atmosphärendruck. Die Dichtlippe wird durch den atmosphärischen Druck, der bei Betrieb der Belüftungs-Pumpe mindestens 2 mbar über dem Küvettenkammer-Innendruck liegt, auf den korrespondierenden zirkulären Schultersitz gedrückt, wodurch die Dichtwirkung der Ringdichtung hergestellt, verbessert bzw. perfektioniert wird. Hierdurch kann praktisch ausgeschlossen werden, dass atmosphärische Luft von außen in den Lüftungskreis eindringt, und auf diese Weise Staub und/oder Luftfeuchtigkeit in den Lüftungskreis eingetragen wird.

Bei abgeschalteter Belüftungs-Pumpe dagegen bildet sich nach einer gewissen Entspannungszeit in der Küvettenkammer atmosphärischer Druck, so dass zwischen der Küvettenkammer und der umgebenden Atmosphäre kein Differenzdruck mehr besteht. Die zirkuläre Dichtlippe liegt dann nur noch lose auf dem zirkulären Schultersitz auf, so dass die Deckelanordnung leicht durch eine entsprechende öffnende Drehbewegung geöffnet werden kann. Da auch während des Schließens der Deckelanordnung die Küvettenkammer ohnehin fluidisch unmittelbar mit der umgebenden Atmosphäre verbunden ist und die Belüftungs-Pumpe beim Schließvorgang abgeschaltet bleibt, liegt während der Schließbewegung der Deckelanordnung die Dichtlippe nur lose auf dem korrespondierenden zirkulären Schultersitz auf, so dass auch bei der Schließbewegung die die Drehbewegung behindernde Haftung zwischen der Dichtlippe und dem Schultersitz gering ist, und die zirkuläre Schließbewegung nur wenig behindert.

Auf diese Weise ist mit relativ einfachen Mitteln ein Wasseranalyse- Gerät geschaffen, dessen die Küvettenkammer abschließende Deckelanordnung durch eine einfache und relativ widerstandsarme Drehbewegung geöffnet bzw. geschlossen werden kann, wenn die Belüftungs-Pumpe abgeschaltet ist.

Grundsätzlich kann der Dichtungskörper an dem Gerätegehäuse und der korrespondierende zirkuläre Schultersitz an dem Dichtungskörper vorgesehen sein. Vorzugsweise ist aber der Dichtungskörper an der Deckelanordnung und ist der zirkuläre Schultersitz an dem Gerätegehäuse vorgesehen.

Vorzugsweise ist die Verschluss-Mimik als Bajonettverschluss ausgebildet. Ein Bajonettverschluss bietet die Möglichkeit, durch eine relativ kleine Drehbewegung von beispielsweise weniger als 100° den Öffnungs- bzw. Schließvorgang auszuführen.

Der Dichtungskörper weist einen Spannring auf, der radial auf eine zylindrische Stützfläche der Deckelanordnung bzw. des Gerätegehäuses aufgespannt ist.

Besonders bevorzugt ist der Spannring von außen auf eine radial innenliegende zylindrische Stützfläche aufgespannt. Auf diese Weise ist die Applikation des Dichtungskörpers auf die zylindrische Stützfläche an dem betreffenden Körper, also an der Deckelanordnung oder an dem Gerätegehäuse, relativ einfach. Insbesondere der Austausch des Dichtungskörpers ist auf diese Weise relativ einfach und bedienungssicher.

Vorzugsweise ist der Schultersitz im Querschnitt gesehen konvex und abgerundet ausgebildet. Der Schultersitz ist im Querschnitt derart geformt, dass die Dichtlippe großflächig bzw. vollflächig an dem Schultersitz anliegen kann und nicht von dem Schultersitz abhebt, auch dann nicht, wenn der Differenzdruck relativ gering ist. Auf diese Weise wird auch langfristig eine hohe Dichtwirkung und Dichtqualität realisiert.

Der Küvettenkammer-Lufteinlass, durch den die Küvettenkammer mit trockener Luft belüftet wird, weist ein pneumatisches Drosselelement auf. Hiermit sind grundsätzlich alle Elemente gemeint, die bei Luft-Durchfluss eine gewisse Drosselwirkung haben und auf diese Weise eine Druckdifferenz zwischen der stromaufwärtigen Seite und der stromabwertigen Seite verursachen. Hierdurch wird zwischen der Belüftungs-Pumpe und dem pneumatischen Drosselelement ein Überdruck aufgebaut und wird stromabwärts des pneumatischen Drosselelements ein Unterdruck aufgebaut, so dass in der Küvettenkammer stets ein Unterdruck herrscht, wenn die Belüftungs-Pumpe in Betrieb ist. Das pneumatische Drosselelement kann beispielsweise eine Membran sein, die zwar luftdurchlässig ist, jedoch einen Wasseraustritt aus der Küvettenkammer verhindert.

Vorzugsweise ist die Meßküvette an der Deckelanordnung befestigt, so dass beim Öffnen und Entfernen der Deckelanordnung von dem Gerätegehäuse auch die Meßküvette aus der Küvettenkammer entfernt wird. Auf diese Weise ist die Meßküvette einfach zu entfernen und zu reinigen. Die Meßküvette kann einfach lösbar an der Deckelanordnung befestigt sein, so dass die Meßküvette einfach von der Deckelanordnung abgenommen und gereinigt bzw. ausgetauscht werden kann. Ferner ist auf diese Weise auch die Küvettenkammer besser zugänglich, nachdem die Deckelanordnung von dem Gerätegehäuse entfernt wurde.

Gemäß einer bevorzugten Ausführungsform ist das Wasseranalyse-Gerät ein Prozess-Trübungsmessgerät. Bei Trübungsmessgeräten ist der Eintrag von Staub in die Küvettenkammer unbedingt zu verhindern, da das optische Nutzsignal einer Trübungsmessung sehr klein ist und schon durch sehr wenig Staub stark verfälscht werden kann. Bei einem Prozessgerät zur Trübungsmessung wird die Wasserprobe kontinuierlich durch die Meßküvette gepumpt. Wenn die Wasserprobe kälter ist als die Luft in der Küvettenkammer, führt dies zu Kondensation von Luftfeuchtigkeit der sich in der Küvettenkammer befindlichen Luft.

In dem Lüftungskreis ist vorzugsweise ein Feuchtigkeits-Absorptionsmittel vorgesehen, durch das der Lüftungskreis-Luft ständig Feuchtigkeit entzogen wird. Durch eine zuverlässige Dichtungsanordnung wird verhindert, dass aus der umgebenden Atmosphäre ständig neue Feuchtigkeit in den Lüftungskreis eingetragen wird, so dass das Erschöpfungsintervall für das Absorptionsmittel sehr lang sein kann.

Im Folgenden wird ein Ausführungsbeispiel der Erfindung anhand der Zeichnungen näher erläutert. Es zeigen:
Figur 1 einen Längsschnitt eines schematisch dargestellten Wasseranalyse-Geräts mit einem Lüftungskreis in einem Gerätegehäuse, das durch eine Deckelanordnung verschlossen ist, und
Figur 2 eine vergrößerte Darstellung der Ringdichtung zwischen der Deckelanordnung und dem Gerätegehäuse.

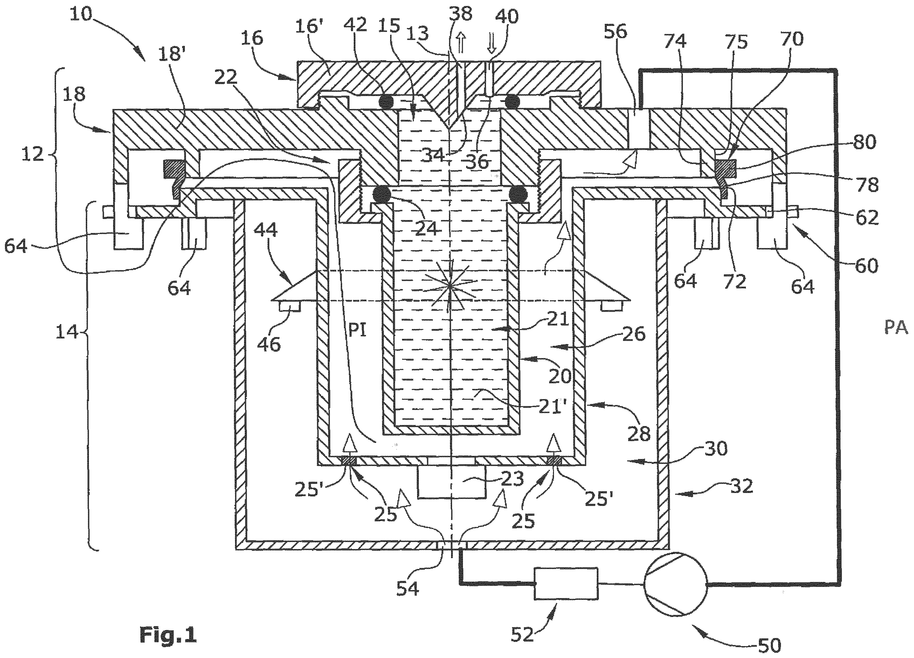

Figur 1 zeigt schematisch ein Wasseranalyse-Gerät 10, das vorliegend als ein Prozess-Trübungsmessgerät ausgebildet ist, und der Messung und Bestimmung der Trübung einer Wasserprobe 21' in dem Küvetteninneren 21 einer transparenten und zylindrischen Meßküvette 20 dient, die vorzugsweise aus transparentem farblosen Glas besteht. Das Wasseranalyse-Gerät 10 ist vorliegend ein Prozess-Gerät, kann jedoch grundsätzlich auch als Labor-Gerät ausgebildet sein. Das Wasseranalyse-Gerät ist insbesondere geeignet für den Einsatz in einer Umgebung mit einer hohen relativen bzw. absoluten Luftfeuchtigkeit.

Das Wasseranalyse-Gerät 10 setzt sich strukturell im Wesentlichen aus einem Gerätegehäuse 14, einer das Gerätegehäuse 14 fluiddicht verschließenden Deckelanordnung 12, einer Belüftungs-Pumpe 50 und einer Trocknungs-Einheit 52 stromabwärts der Belüftungs-Pumpe 50 zusammen, die außerhalb des Gerätegehäuses 14 angeordnet sind.

In dem Gehäuseinneren weist das Wasseranalyse-Gerät 10 eine durch ein becherförmiges Küvettenkammer-Gehäuse 28 definierte Küvettenkammer 26 auf, in der die Meßküvette 20 angeordnet ist, und weist eine Meßkammer 30 auf, die das Küvettenkammer-Gehäuse 28 außen umgibt und ihrerseits durch ein becherförmiges Meßkammer-Gehäuse 32 außen begrenzt ist. Sowohl das Meßkammer-Gehäuse 32 als auch das Küvettenkammer-Gehäuse 28 als auch die Meßküvette 20 sind becherförmig ausgebildet.

Das Wasseranalyse-Gerät 10 weist einen Lüftungskreis zum Belüfteten des Gehäuseinneren auf, um in der Meßkammer 30 und in der Küvettenkammer 26 an den dortigen Oberflächen Kondensation von Feuchtigkeit zu verhindern.

Das Prozess-Wasseranalyse-Gerät 10 ist mit einem Probeneinlass 40 und einem Probenauslass 38 ausgestattet, durch die die Wasserprobe in das Küvetteninnere 21 der Meßküvette 20 kontinuierlich oder nichtkontinuierlich einfließt bzw. aus dieser wieder herausfließt. Die Meßküvette 20 ist innerhalb der Küvettenkammer 26 angeordnet, die durch den Lüftungskreis kontinuierlich mit getrockneter Luft belüftet wird.

Die Küvettenkammer 26 wird im Wesentlichen durch das becherförmige transparente Küvettenkammer-Gehäuse 28 gebildet und durch die Deckelanordnung 12 abgeschlossen. Die Meßküvette 20 ist an der Deckelanordnung 12 hängend und durch eine Flanschmutter 22 lösbar befestigt. Zwischen dem Öffnungskragen der Meßküvette 20 und dem ringförmigen Flansch an dem Hauptkörper 18 der Deckelanordnung 12 ist ein ringförmiger Dichtring 24 angeordnet, der eine fluiddichte Abdichtung in diesem Bereich sicherstellt.

Die Trübung einer Flüssigkeit ist ein Maß für die Konzentration fester Partikel in der Wasserprobe 21'. Die Trübung wird durch axiale Einleitung eines Lichtstrahls in die Meßküvette 20, der von einer Lichtquelle 23 axial entlang einer optischen Längsachse 13 emittiert wird, und durch die Messung der Lichtintensität des von der Wasserprobe 21' gestreuten Lichtstrahl- Lichts in einem Winkel von 90° im Verhältnis zu der Längsachse 13 gemessen, wobei das Streulicht durch ein ringförmiges optisches Element 44 gesammelt und einem optischen Trübungssensor 46 zugeführt wird. Das ringförmige optische Element 44 und der Trübungssensor 46 sind in der Meßkammer 30 angeordnet.

Die Deckelanordnung 12 besteht im Wesentlichen aus einem ringförmigen Hauptdeckel 18 mit einer Zentralöffnung 15 und einem die Zentralöffnung 15 verschließenden Zentraldeckel 16, der mit dem Hauptdeckel 18 verschraubt ist. Der Zentraldeckel 16 wird von einem Deckelkörper 16' aus opakem Kunststoff gebildet, und weist außenseitig den Probeneinlass 40 und den Probenauslass 38 auf. Der außenseitige Probeneinlass 40 führt über eine Leitung in dem Deckelkörper 16' zu einem innenseitigen Probeneinlass 36, durch den die Wasserprobe in die Zentralöffnung 15 und in das Küvetteninnere 21 einströmt. Der außenseitige Probenauslass 38 führt über eine Leitung in dem Deckelkörper 16' zu einem innenseitigen proben Auslass 34, durch den die Wasserprobe aus dem Küvetteninneren 21 durch die Zentralöffnung 15 ausströmt. Zwischen dem Hauptdeckel 18 und dem Zentraldeckel 16 ist eine axiale Ringdichtung 42 vorgesehen, die eine fluiddichte Abdichtung des Innenraums des Küvetten Innenraums des Küvetteninneren 21 gegenüber der außenseitigen Atmosphäre sicherstellt.

Der Hauptdeckel 18 besteht aus einem komplexen Hauptdeckel-Körper 18' aus opakem Kunststoff, und weist insbesondere eine Verschluss-Mimik 60 und eine Ringdichtung 70 auf, die beide jeweils mit entsprechenden Komponenten an dem Gerätegehäuse 14 funktional zusammenwirken.

Die Verschluss-Mimik 60 ist vorliegend als Bajonettverschluss ausgebildet, und weist deckelseitig mehrere axiale Bajonettzähne 64 auf, die in der in Fig. 1 dargestellten verriegelten Schließposition jeweils einen korrespondierenden gehäuseseitigen Bajonettsteg 62 hintergreifen. Die Verschluss-Mimik 60 ist derart ausgebildet, dass die Öffnungs- bzw. Schließbewegung lediglich einen Winkel von 15° bis 30° ausmacht, besonders bevorzug von 20°.

Der Lüftungskreis weist die elektrische Belüftungs-Pumpe 50, die sich stromabwärts daran anschließende passive Trocknungs-Einheit 52 mit Molekularsieb, die topfförmige Meßkammer 30, in die die von der Trocknung-Einheit 52 kommende Luft durch eine Geräte-Einlassöffnung 54 im Gehäuse-Boden einströmt, die Küvettenkammer 26, in die die von der Meßkammer 30 kommende Luft durch mehrere Luft-Einlassöffnungen 25 im Boden des topfförmigen Küvettenkammer-Gehäuses 28 einströmt, und einen Geräte-Luftauslass 56 auf, von wo die Trocknungs-Luft über eine Leitung zu der Belüftungs-Pumpe 50 fließt.

In den im Boden des Küvettenkammer-Gehäuses 28 vorgesehenen Luft-Einlassöffnungen 25 sind jeweils pneumatische Drosselelemente 25' vorgesehen, die von luftdurchlässigen Membranen gebildet werden, die jedoch nicht durchlässig für Wassermoleküle sind. Die Drosselelemente 25' sorgen bei laufender Belüftungs-Pumpe 50 für einen Druckabfall im ein- bis niedrigen dreistelligen Millibar-Bereich, so dass in der Meßkammer 30 gegenüber dem Atmosphärendruck PA ein gewisser Überdruck und in der Küvettenkammer 26 gegenüber der Atmosphäre ein gewisser Unterdruck herrscht. In jedem Fall ist bei laufender Belüftungs-Pumpe 50 der Küvettenkammer-Druck PI um mindestens einige Millibar niedriger als der atmosphärische Druck PA außerhalb des Wasseranalyse-Geräts 10.

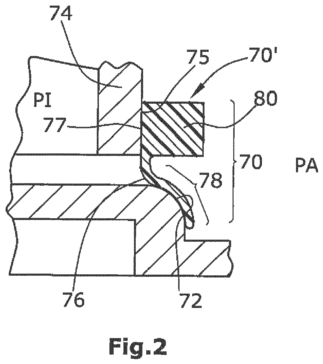

Die Ringdichtung 70 ist in der Figur 2 detailliert dargestellt. Sie wird im Wesentlichen von einem deckelseitigen axialen Ringsteg 74, einem an dem Ringsteg 74 fixierten Dichtungskörper 70' mit einer zirkulären Dichtlippe 78 und einem gehäuseseitigen zirkulären und korrespondierenden Schultersitz 72 gebildet, auf dem die flexible Dichtlippe 78 mit ihrer proximalen Lippenfläche 76 aufliegt. Der Schultersitz 72 ist im Querschnitt konvex und abgerundet ausgebildet, wie insbesondere in Figur 2 gut erkennbar ist. Die Abrundung ist im Radius so groß, dass die Dichtlippe 78 überall vollflächig auf dem Schultersitz 72 aufliegt.

Der elastische Dichtungskörper 70' besteht im Wesentlichen aus einem Spannring 80, der mit seiner proximalen Innenumfangsfläche 77 auf eine korrespondierenden zylindrischen distalen Stützfläche 75 des Ringstegs 74 aufgeschoben und aufgespannt ist, und der schmallippigen Dichtlippe 78. Es ist im Bereich der Ringdichtung 70 keinerlei Fett zur Verbesserung der Dichtwirkung vorgesehen, die Ringdichtung 70 ist also absolut fettfrei.

Solange die Belüftungs-Pumpe 50 läuft, herrscht in der Küvettenkammer 26 gegenüber dem atmosphärischen Druck PA ein Unterdruck, der praktisch identisch auch im Bereich des zirkulären Schultersitzes 72 an der proximalen Lippenfläche 76 der Dichtlippe 78 herrscht. Hierdurch wird die Dichtlippe 78-und vollflächig auf den Schultersitz 72 gedrückt, wodurch sich hier eine sehr gute fluidische Abdichtung ergibt.

Wenn die Belüftungs-Pumpe 50 nicht mehr läuft, stellt sich nach einer gewissen Zeit in der Küvettenkammer 26 mehr oder weniger atmosphärischer Druck ein, so dass die Dichtlippe 78 nur noch lose auf der auf dem Schultersitz 72 aufliegt. Hierdurch besteht dann auch keine nennenswerte Haftreibung mehr zwischen der Dichtlippe 78 und dem Schultersitz 72, so dass die Deckelanordnung 12 problemlos ergriffen, zum Öffnen in Öffnungs-Drehrichtung gedreht und schließlich von dem Gerätegehäuse 14 abgenommen werden kann.

Während des Schließvorgangs, also wenn die Deckelanordnung 12 auf das Gerätegehäuse 14 aufgesetzt und in Schließrichtung verdreht wird, ist die Belüftungs-Pumpe 50 abgeschaltet, so dass auch während des Schließvorgangs sichergestellt ist, dass die Dichtlippe 78 nur lose auf dem zirkulären Schultersitz 72 aufliegt und nur geringe Reibungskräfte auftreten. Sobald der Schließvorgang abgeschlossen ist, kann die Belüftungs-Pumpe 50 eingeschaltet werden, so dass in der Küvettenkammer 26 ein Unterdruck generiert wird, durch den sich die Dichtlippe 78 auf dem Schultersitz 72 festsaugt.

Sollte während des Meßbetriebs eine Undichtigkeit im Bereich der Ringdichtung 24 auftreten oder aber die Meßküvette 20 undicht werden, kann die mit Überdruck in die Küvettenkammer 26 einfließende Flüssigkeit durch die Ringdichtung 70 problemlos abfließen, da dann der Meßkammer-Innendruck PI größer ist als der atmosphärische Druck PA. Auf diese Weise wird verhindert, dass die Meßproben-Flüssigkeit in andere Teile des Wasseranalyse-Geräts 10 fließt und dort Beschädigungen und Zerstörungen auslösen kann.

## Patentansprüche

1. Wasseranalyse-Gerät (10) mit einer transparenten Meßküvette (20), einer Lichtquelle (23) und einem Licht-Detektor (46) zur Erfassung eines optischen Parameters einer Wasserprobe (21') in der transparenten Meßküvette (20), einer Küvettenkammer (26), in der die Meßküvette (20) angeordnet ist,
einem Lüftungskreis zum Belüften der Küvettenkammer (26), wobei der Lüftungskreis die Küvettenkammer (26), eine Belüftungs-Pumpe (50) stromabwärts der Küvettenkammer (26) und einen Küvettenkammer-Lufteinlass (25) stromabwärts der Belüftungs-Pumpe (50) aufweist, wobei der Küvettenkammer-Lufteinlass ein pneumatisches Drosselelement (25') aufweist, so dass bei Betrieb der Belüftungs-Pumpe (50) in der Küvettenkammer (26) gegenüber der Atmosphäre ein Unterdruck herrscht, und einer Deckelanordnung (12) und einem Gerätegehäuse (14), wobei das Gerätegehäuse (14) die Küvettenkammer (26) bildet, die durch die Deckelanordnung (12) fluidisch verschlossen ist, **dadurch gekennzeichnet,**
**dass** der Unterdruck ein Differenzdruck von mindestens 2,0 mbar ist,
**dass** die Deckelanordnung (12) und das Gerätegehäuse (14) eine Drehverschluss-Mimik (60) aufweisen, so dass die Deckelanordnung (12) durch eine Drehbewegung an dem Gerätegehäuse (14) verriegelt bzw. entriegelt werden kann,
**dass** die Deckelanordnung (12) und das Gerätegehäuse (14) eine kreisringförmige und zu der Drehbewegung koaxiale Ringdichtung (70) bilden, wobei die Ringdichtung (70) von einem an der Deckelanordnung (12) bzw. an dem Gerätegehäuse (14) vorgesehenen elastischen Dichtungskörper (70') mit einer zirkulären Dichtlippe (78) und einem an dem Gerätegehäuse (14) bzw. an der Deckelanordnung (12) vorgesehenen korrespondierenden zirkulären Schultersitz (72) gebildet wird, auf den die Dichtlippe (78) durch den atmosphärischen Differenzdruck gedrückt wird,
und **dass** der Dichtungskörper (70') einen Spannring (80) aufweist, der radial auf eine zylindrische Stützfläche (75) der Deckelanordnung (12) bzw. des Gerätegehäuses (14) aufgespannt ist.

2. Wasseranalyse-Gerät (10) nach Anspruch 1, **dadurch gekennzeichnet, dass** die Drehverschluss-Mimik (60) als Bajonettverschluss ausgebildet ist.

3. Wasseranalyse-Gerät (10) nach einem der vorangegangenen Ansprüche, **dadurch gekennzeichnet, dass** der Schultersitz (72) im Querschnitt konvex und abgerundet ausgebildet ist.

4. Wasseranalyse-Gerät (10) nach einem der vorangegangenen Ansprüche, wobei die Meßküvette (20) an der Deckelanordnung (12) befestigt ist.

5. Wasseranalyse-Gerät (10) nach einem der vorangegangenen Ansprüche, wobei das Wasseranalyse-Gerät (10) ein Prozess-Trübungsmessgerät ist.

## Claims

1. Water analysis apparatus (10) with a transparent measuring cuvette (20), a light source (23) and a light detector (46) for acquiring an optical parameter of a water probe (21') in the transparent measuring cuvette (20), a cuvette chamber (26), in which the measuring cuvette (20) is arranged, a ventilation circuit for ventilating the cuvette chamber (26), wherein the ventilation circuit has the cuvette chamber (26), a ventilation pump (50) downstream of the cuvette chamber (26) and a cuvette chamber air inlet (25) downstream of the ventilation pump (50), wherein the cuvette chamber air inlet has a pneumatic throttling element (25'), so that when the ventilation pump (50) is operated, there is a vacuum in the cuvette chamber (26) in relation to the atmosphere, and a cover arrangement (12) and an apparatus housing (14), wherein the apparatus housing (14) forms the cuvette chamber (26) that is closed fluidly by the cover arrangement (12), **characterized in that**
the vacuum is a differential pressure of at least 2.0 mbar,
the cover arrangement (12) and the apparatus housing (14) have a screw top mimic (60), so that the cover arrangement (12) can be bolted to or unbolted from the apparatus housing (14) through a rotational movement, the cover arrangement (12) and the apparatus housing (14) form a circular ring-shaped ring seal (70) that is coaxial with the rotational movement, wherein the ring seal (70) is formed by an elastic seal body (70') provided on the cover arrangement (12) or on the apparatus housing (14) with a circular seal lip (78) and a corresponding circular shoulder seat (72) provided on the apparatus housing (14) or on the cover arrangement (12) onto which the seal lip (78) is pressed by the atmospheric differential pressure,
and the seal body (70') has a blocking ring (80), which is fixed radially to a cylindrical support surface (75) of the cover arrangement (12) or of the apparatus housing (14).

2. Water analysis apparatus (10) according to claim 1, **characterized in that** the screw top mimic (60) is configured as a bayonet lock.

3. Water analysis apparatus (10) according to one of the preceding claims, **characterized in that** the shoulder seat (72) in the cross section is configured convexly and rounded.

4. Water analysis apparatus (10) according to one of the preceding claims, wherein the measuring cuvette (20) is fixed to the cover arrangement (12).

5. Water analysis apparatus (10) according to one of the preceding claims, wherein the water analysis apparatus (10) is a process turbidity meter.

## Revendications

1. Dispositif d'analyse d'eau (10) avec une cuvette de mesure transparente (20), une source de lumière (23) et un détecteur de lumière (46) pour la détection d'un paramètre optique d'un échantillon d'eau (21') dans la cuvette de mesure transparente (20), une chambre de cuvette (26), dans laquelle la cuvette de mesure (20) est agencée, un circuit d'aération pour aérer la chambre de cuvette (26), dans lequel le circuit d'aération comporte la chambre de cuvette (26), une pompe d'aération (50) en aval de la chambre de cuvette (26) et un conduit d'entrée d'air de la chambre de cuvette (25) en aval de la pompe d'aération (50), dans lequel le conduit d'entrée d'air de la chambre de cuvette comporte un élément d'étranglement pneumatique (25'), de sorte que lors du fonctionnement de la pompe d'aération (50) dans la chambre de cuvette (26) une dépression règne par rapport à l'atmosphère, et un ensemble couvercle (12) et un boîtier de dispositif (14), dans lequel le boîtier de dispositif (14) forme la chambre de cuvette (26), qui est fermée par l'ensemble couvercle (12) de manière fluidique, **caractérisé en ce que**
la dépression est une pression différentielle d'au moins 2,0 mbar,
l'ensemble couvercle (12) et le boîtier de dispositif (14) comportent un dispositif de fermeture par rotation (60), de sorte que l'ensemble couvercle (12) peut être verrouillé ou déverrouillé sur le boîtier de dispositif (14) par un mouvement de rotation,
l'ensemble couvercle (12) et le boîtier de dispositif (14) forment un joint d'étanchéité annulaire (70) en forme d'anneau circulaire et coaxial au mouvement de rotation, le joint d'étanchéité annulaire (70) étant formé par un corps d'étanchéité élastique (70') avec une lèvre d'étanchéité circulaire (78) et prévu sur l'ensemble couvercle (12) ou sur le boîtier de dispositif (14), et un siège d'épaulement circulaire (72) correspondant, prévu sur le boîtier de dispositif (14) ou sur l'ensemble couvercle (12), sur lequel la lèvre d'étanchéité (78) est pressée par la pression différentielle atmosphérique,
et le corps d'étanchéité (70') comporte un anneau de serrage (80), qui est fixé radialement sur une surface de support cylindrique (75) de l'ensemble couvercle (12) ou du boîtier de dispositif (14).

2. Dispositif d'analyse d'eau (10) selon la revendication 1, **caractérisé en ce que** le dispositif de fermeture par rotation (60) est réalisé en forme de verrouillage par baïonnette.

3. Dispositif d'analyse d'eau (10) selon l'une des revendications précédentes, **caractérisé en ce que** le siège d'épaulement (72) dans la section transversale est réalisé de forme convexe et arrondie.

4. Dispositif d'analyse d'eau (10) selon l'une des revendications précédentes, dans lequel la cuvette de mesure (20) est fixée à l'ensemble couvercle (12).

5. Dispositif d'analyse d'eau (10) selon l'une des revendications précédentes, dans lequel le dispositif d'analyse d'eau (10) est un turbidimètre de process.
